# EUROPÄISCHE PATENTANMELDUNG

(11) **EP 4 684 760 A1**
(43) Veröffentlichungstag der Anmeldung: **28.01.2026**
(21) Anmeldenummer: 25181232.7
(22) Anmeldetag: 06.06.2025
(51) Int. Cl.: A61F 2/18

(54) **AUS EINEM GEBINDE AUSWÄHLBARE GEHÖRKNÖCHELCHENPROTHESE**

(30) Priorität: 25.07.2024 DE 102024121204
(71) Anmelder: Heinz Kurz GmbH, 72144 Dusslingen (DE)
(72) Erfinder: SANDER, Alexander, 79822 Titisee-Neustadt (DE); STIEGELE, Thomas, 72585 Riederich (DE); MERTENS, Matthias, 24568 Kaltenkirchen (DE)
(74) Vertreter: Kohler Schmid Möbus Patentanwälte

(57) **Zusammenfassung**

Eine Gehörknöchelchenprothese (20) mit einem als Kopfplatte zur Anlage am Trommelfell ausgebildeten erstes Befestigungselement (21), einem zweiten Befestigungselement (22) zur mechanischen Verbindung mit einem Glied der Gehörknöchelchen-Kette oder mit dem Innenohr sowie einem entlang einer Längsachse die beiden Befestigungselemente Schall-leitend miteinander verbindenden, Schaft-förmigen Verbindungselement (24), wobei das erste Befestigungselement in seinem Zentrum eine zur Fixierung verengbare Durchgangsbohrung (25) zur Aufnahme des Verbindungselements aufweist, ist dadurch gekennzeichnet, dass das Verbindungselement durch die Durchgangsbohrung hindurch auf die dem zweiten Befestigungselement gegenüberliegende Seite des ersten Befestigungselements ragt und an seinem dem zweiten Befestigungselement gegenüberliegenden freien Ende ein drittes Befestigungselement (23) trägt, welches zur Verbindung mit einem Glied der Gehörknöchelchen-Kette oder mit dem Innenohr ausgestaltet ist, und dass das Verbindungselement in seinen beiden an die Durchgangsbohrung angrenzenden Bereichen so gestaltet ist, dass es oberhalb oder unterhalb des ersten Befestigungselements leicht abgetrennt werden kann. Dieses Gebinde bietet dem Mittelohr-Chirurgen unmittelbar vor dem Einsetzen einer ausgewählten Prothese ins Mittelohr des Patienten eine simple und schnelle Auswahlmöglichkeit aus einer Reihe von unterschiedlichen Prothesenarten.

## Beschreibung

Die Erfindung betrifft eine Gehörknöchelchenprothese, die mindestens ein Glied oder Teile eines Gliedes der Gehörknöchelchenkette ersetzt oder überbrückt, wobei die Gehörknöchelchenprothese ein als flache Kopfplatte zur mechanischen Anlage am Trommelfell ausgebildetes erstes Befestigungselement, ein zweites Befestigungselement zur mechanischen Verbindung mit einem Glied oder Teilen eines Gliedes der Gehörknöchelchen-Kette oder mit dem Innenohr sowie ein entlang einer Längsachse die beiden Befestigungselemente Schall-leitend miteinander verbindendes, Schaft-förmiges Verbindungselement umfasst, und wobei das Platten-förmige erste Befestigungselement in seinem Zentrum eine Durchgangsbohrung zur Aufnahme des Schaft-förmigen Verbindungselements aufweist, die zur Fixierung des Verbindungselements verengbar gestaltet ist.

Eine derartige Vorrichtung ist bekannt aus DE 298 19 892 U1 ≈ EP 0 998 884 B1 ≈ US 6,387,128 B1 (=Referenz [1]).

### Hintergrund der Erfindung

Gehörknöchelchenprothesen werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell beziehungsweise am Hammergriff anliegt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten GehörknöchelchenProthesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können. (Siehe zum Beispiel DE 42 10 235 C1 oder EP 0 809 982 B1 (=Referenz [2])).

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse post-operative Mobilität aufweist, so dass sich die Kopfplatte postoperativ selbstständig der Position des Trommelfells angleichen kann.

Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht starr ausgebildet sind, sondern eine gewisse Flexibilität oder Variabilität aufweisen.

Um eine solche Flexibilität/Variabilität zu erreichen sind verschiedene Befestigungs- und Ankopplungsvorrichtungen für Gehörknöchelchen, die elastische Teile und/oder Gelenke aufweisen, bekannt. Eine solche gelenkige Verbindung zwischen einem an der Steigbügelfußplatte montierbaren Befestigungselement und dem länglichen Schaft ist beispielsweise in der EP 1 181 907 B1 (=Referenz [3]) beschrieben und wird von der Anmelderin unter dem Markennamen "Ball-Joint" angeboten.

Eine weitere, verschiedentlich auftretende Komplikation entsteht durch eine Unterbelüftung des Mittelohrraumes und damit einhergehende Entzündungen, Tumorbildungen, Verwachsungen im Trommelfellbereich sowie Versteifungen desselben. Beispielsweise bei einer Dysfunktion der Eustachischen Röhre kann es im Mittelohr zu einem Unterdruck kommen, der eine Auskrempung bzw. Ausstülpung (sogenannte Retraktion) des Trommelfells und in der Folge eine Verwachsung etwa mit dem Steigbügel bewirken kann. Um dem entgegen zu wirken und postoperativen Bewegungen des Trommelfells folgen zu können, werden die Kopfplatten bei bekannten Gehörknöchelchenprothesen verkippbar relativ zu dem Verbindungselement gestaltet, das die Kopfplatte mit dem zweiten Befestigungselement verbindet uns meistens als länglicher Schaft ausgeführt ist. Eine solche in sich starre, aber gegenüber dem Verbindungselement verkippbare Kopfplatte ist u.a. beschrieben in der US 2004/0162614 A1 (=Referenz [4]).

Während Referenz [3] eine Gehörknöchelchenprothese, bei welcher die Kopfplatte ein eingebautes, mit dem Verbindungsschaft der beiden Befestigungselemente verbundenes Kugelgelenk aufweist, offenbart EP 1 833 424 B1 (=Referenz [5]) sogar einen als Kugelkette ausgebildeten Verbindungsschaft. Dadurch kann die endgültige axiale Länge der Prothese zumindest abgestuft durch die Auswahl einer bestimmten Anzahl an Kugeln in der Kette und Abschneiden der überzähligen Kugeln festgelegt werden.

Nachteilig bei diesen bekannten Gehörknöchelchenprothesen ist allerdings, dass durch die starre Verkippung der Kopfplatte bei lokalen Medialbewegungen des Trommelfells gleichzeitig die gegenüber liegende Seite der Kopfplatte lateral nach außen bewegt wird, wodurch Druckspitzen auf das Trommelfell erzeugt werden.

Die EP 1 972 307 B1 (=Referenz [6]) offenbart eine Gehörknöchelchenprothese mit hochflexibel gestalteter Kopfplatte, bei welcher einerseits die Vorteile der Prothese gemäß der oben genannten Referenz [5], andererseits aber auch die Vorteile der Referenz [4] beschriebenen Prothesen erhalten bleiben, wobei jedoch die gemeinsamen Nachteile einer starren Verkippung der Kopfplatte vermieden werden. Problematisch bei der Gehörknöchelchenprothese nach Referenz [6] ist allerdings ihre Einführung ins Mittelohr des Patienten, weil insbesondere in radialer Richtung bezüglich ihrer Längsachse die Kopfplatte erheblich seitlich ausragt und nur mittels einer großen künstlichen Öffnung operativ durch den TrommelfellBereich in das Mittelohr eingesetzt werden kann. Diese große Öffnung wächst dann post-operativ natürlich auch schwerer wieder zu und hinterlässt zudem entsprechend große Narben.

Zwar umfasst die Kopfplatte bei Referenz [6] flexible Stegelemente, welche innerhalb der Kopfplatten-Ebene verlaufen und einen radial äußeren Ringbereich der Kopfplatte mit einem radial in der Mitte der Kopfplatte angeordneten zentralen Ankoppelbereich verbinden. Diese Stegelemente sind -zusammen mit dem radial äußeren Ringbereich und dem zentralen Ankoppelbereich- fester Bestandteil der in Referenz [6] offenbarten Kopfplatte. Sie sind geometrisch so gestaltet, dass sie bei lokalen Medialbewegungen des Trommelfells einer solchen Medialbewegung -ebenfalls lokal- folgen, jedoch die Bewegung nicht an entfernte Bereiche der Kopfplatte weitergeben.

Für eine etwaige radiale Stauchung der Kopfplatte innerhalb der Kopfplatten-Ebene und damit einer radialen Verkleinerung des Kopfplatten-Durchmesser, gegebenenfalls zum Zwecke einer erleichterten Durchführung durch das Trommelfell, sind die Stegelemente aber nicht ausgebildet, sondern wären für eine derartige Anwendung ungeeignet. Dies ist insbesondere deswegen so, weil der radial äußere Ringbereich starr ausgeführt ist und daher dem Versuch einer solchen radialen Stauchung nicht folgen oder sich höchstens unkontrollierbar verbiegen würde.

Auch für eine Längenveränderung in Richtung der z-Achse ist die in Referenz [6] beschriebene Gehörknöchelchenprothese absolut ungeeignet, da das Verbindungselement, welches längs der z-Achse verläuft und die Kopfplatte mit dem zweiten Befestigungselement starr verbindet, zwar Schall-übertragend, aber als solches steif ausgebildet ist. Eine Flexibilität der Länge des Verbindungselements in z-Richtung ist bei der Gehörknöchelchenprothese nach Referenz [6] ausgeschlossen und auch gar nicht gewünscht.

Die EP 3 311 773 B1 (=Referenz [7]) beschreibt demgegenüber eine Gehörknöchelchenprothese mit regenschirmartig faltbarer Kopfplatte, mittels welcher die Prothese nahezu minimal-invasiv durch eine kleine Öffnung im Trommelfell operativ ins Mittelohr einsetzbar ist.

Ein weiteres wichtiges Thema bei der Implantation von Gehörknöchelchenprothesen ist die Einstellung der korrekten, an die individuellen Gegebenheiten und Geometrieverhältnisse im Mittelohr des Patienten optimal angepassten axialen Länge der Prothese.

Bereits die oben diskutierte Referenz [5] schlägt dazu eine Kugelkette mit abtrennbaren Endkugeln vor. Dies ermöglicht aber leider keine kontinuierliche, sondern lediglich eine stufenweise Längeneinstellung.

Eine längenvariable Gehörknöchelchenprothese mit einem im Verbindungselement zwischen dem ersten und dem zweiten Befestigungselement eingebauten Verschiebemechanismus zur stufenlosen Längeneinstellung ist in der DE 10 2007 041 539 B4 (=Referenz [8]) beschrieben.

Anstelle einer solchen -in der Herstellung relativ aufwändigen-Verschiebemechanik schlägt die EP 2 238 946 B1 (=Referenz [9]) eine längenvariable Gehörknöchelchenprothese vor, bei welcher im Verbindungselement eine Ziehharmonika-artige Struktur eingebaut ist. Die axiale Länge der Gehörknöchelchenprothese kann dann durch eine axiale Stauchung dieser Struktur verkürzt und durch ein Auseinanderziehen derselben vergrößert werden.

In der EP 2 601 909 B1 (=Referenz [10]) wiederum ist zur axialen Längeneinstellung der Gehörknöchelchenprothese eine Verschiebemechanik im Verbindungselement vorgesehen, welche ein Aufnahmeteil und einen das Aufnahmeteil mit zwei Schenkeln klammerartig umgreifendes Einschiebeteil umfasst, wobei das Aufnahmeteil und das Einschiebeteil relativ zueinander in axialer Richtung des Verbindungselements verfahrbar sind.

Eine Laser-aktivierbare längenvariable Gehörknöchelchenprothese ist in der EP 3 130 315 B1 (=Referenz [11]) offenbart. Darin wird vorgeschlagen, das Verbindungselement mit streckbaren und/oder stauchbaren, schlaufenartig gefalteten Teilsträngen aus einem Material mit Formgedächtnis aufzubauen. Mit den Schlaufen dieser Teilstränge sind Aktivierungsflächen wärmeleitend verbunden, welche durch Hitzeeinwirkung eine thermische Aktivierung und damit eine Verformung der Schlaufen bewirken können. Auf diese Weise kann die axiale Länge der Gehörknöchelchenprothese verändert und nach Wunsch eingestellt werden.

Die EP 1 961 400 B1 (=Referenz [12]) beschreibt eine partielle Gehörknöchelchenprothese, an deren einem Ende eine Schlinge vorgesehen ist, mittels welcher ein Bereich eines Gliedes der Gehörknöchelchen-Kette kraftschlüssig umgriffen werden kann, wobei die Schlinge so ausgebildet ist, dass sie im implantierten Zustand der Prothese zwar fest, aber nur in Teilbereichen an diesem Glied anliegt, um eine vollständige Strangulierung der Ankoppelstelle am Gehörknöchelchen zu vermeiden. Zudem wird in Referenz [12] vorgeschlagen, zumindest eines der Befestigungselemente aus einem Material mit Formgedächtnis (=memory effect) oder superelastischen Eigenschaften, vorzugsweise aus Nitinol herzustellen.

In der DE 10 2022 119 451 B3 (=Referenz [13]) wird eine Gehörknöchelchenprothese mit in-situ-Elongation vorgestellt, bei welcher die Prothese zweigeteilt aufgebaut ist, nämlich aus einer Kopfplatte einerseits und aus dem Verbindungselement mit einem zweiten Befestigungselement andererseits. Die Kopfplatte weist dabei Klammer-artige Arme auf, mittels denen sie an Rillen angekoppelt werden kann, die azimutal um das Verbindungselement herum verlaufen. Damit kann dann auch nach erfolgter Implantation eine Längeneinstellung der Gehörknöchelchenprothese vorgenommen werden.

Aus der DE 10 2009 006 047 B3 (=Referenz [14]) ist eine passive Gehörknöchelchen-prothese mit Applikator bekannt, die sich dadurch auszeichnet, dass ein länglicher Applikator zum Transport der Gehörknöchelchenprothese von einer Sterilverpackung zum Operationsfeld und Einbringen ins Mittelohr oder den Gehörgang vorgesehen ist, welcher einerseits ein von der Gehörknöchelchenprothese weg ragendes freies Ende zur Handhabung sowie andererseits einen Eingriffsteil aufweist, der zunächst kraftschlüssig, formschlüssig oder über eine abbrechbare oder abscherbare Materialbrücke an der Gehörknöchelchenprothese befestigt ist, und nach dem Einsetzen der Gehörknöchelchenprothese ins Mittelohr oder den Gehörgang von der Gehörknöchelchenprothese abgetrennt und mitsamt dem Applikator aus dem Mittelohr oder dem Gehörgang entfernt werden kann. Dies ermöglicht unaufwändig und kostengünstig die Bereitstellung einer aus der sterilen Verpackung bereits operationsfertig entnehmbaren Einheit aus einer Gehörknöchelchenprothese und einem optimal auf deren Geometrie angepassten, individuellen Transportmittel. Der Applikator kann nach der Implantation der Prothese problemlos aus dem Mittelohr entfernt und entsorgt werden, was die praktische Handhabung der erfindungsgemäßen Gehörknöchelchenprothese für den Chirurgen enorm erleichtert. Der gesamte Vorgang ist standardisiert und außer dem Operationsmikroskop sind keine weiteren Hilfsmittel erforderlich, insbesondere keine sterile Vorbehandlung derselben.

Die DE 10 2020 108 887 A1 (=Referenz [15]) beschreibt eine längeneinstellbare Gehörknöchelchenprothese mit einer als elliptischer Ring gestalteten Trommelfell-Kopfplatte mit Verriegelungselement, welches an unterschiedlichen axialen Positionen des schaftförmigen Verbindungselements die Kopfplatte fest am Verbindungselement verriegeln kann.

In der US 2020/0113675 A1 (=Referenz [16]) schließlich wird eine Mittelohrprothese mit einer mit biokompatiblem Silikon beschichteten Trommelfell-Kopfplatte gezeigt, bei der die Beschichtung in der Kopfplatte derart integriert ist, dass sie im implantierten Zustand der Prothese zumindest teilweise das Trommelfell berührt.

Die Druckschriften der Schwesterpatente zur bereits oben zitierten Referenz [1] offenbaren demgegenüber eine gattungsgemäße Gehörknöchelchenprothese mit sämtlichen eingangs definierten Merkmalskomplexen. Insbesondere wird darin vorgeschlagen, im Verbindungselement der generischen Gehörknöchelchenprothese streifenartige Stegelemente vorzusehen, die radial von der Längsachse weg nach außen abgespreizt werden können und dabei die axiale Länge des Verbindungselements und damit die axiale Länge der Gehörknöchelchenprothese verkürzen. Die Spreizung der Stegelemente erfolgt von der Seite her, also in radialer Richtung bezüglich des Verbindungselements.

### Aufgabe der Erfindung

Aufgabe der vorliegenden Erfindung ist es demgegenüber, eine gattungsgemäße Gehörknöchelchenprothese der eingangs definierten Art mit möglichst einfachen technischen Mitteln dahingehend zu verbessern, dass unaufwändig und kostengünstig eine simple und schnelle Auswahlmöglichkeit für den Mittelohr-Chirurgen aus einer Reihe von unterschiedlichen Arten von Gehörknöchelchenprothesen unmittelbar vor dem Einsetzen der vom Chirurgen konkret ausgewählten Prothese ins Mittelohr des Patienten zu schaffen.

Eine derartige Auswahlmöglichkeit ist in keiner einzigen der oben diskutierten Referenzen [1] bis [13] vorgesehen oder auch nur angedeutet.

### Kurze Beschreibung der Erfindung

Erfindungsgemäß wird diese Aufgabe auf ebenso überraschend einfache wie wirkungsvolle Art und Weise dadurch gelöst, dass das Schaft-förmige Verbindungselement durch die Durchgangsbohrung hindurch auf die dem zweiten Befestigungselement gegenüberliegende Seite des ersten Befestigungselements ragt, dass das Verbindungselement an seinem dem zweiten Befestigungselement gegenüberliegenden freien Ende ein drittes Befestigungselement trägt, welches zur mechanischen Verbindung mit einem Glied oder Teilen eines Gliedes der Gehörknöchelchen-Kette oder mit dem Innenohr ausgestaltet ist, und dass das Schaft-förmige Verbindungselement zumindest in seinen beiden an die Durchgangsbohrung des ersten Befestigungselements beiderseits angrenzenden Bereichen so gestaltet ist, dass es oberhalb oder unterhalb des Platten-förmigen ersten Befestigungselements abgebrochen oder mittels eines Separations-Werkzeugs abgetrennt werden kann.

Dadurch können auf relativ simple Weise die Vorteile der oben beschriebenen gattungsgemäßen Gehörknöchelchenprothese, wie sie in Referenz [1] beschrieben ist, genutzt werden, wobei jedoch nunmehr der Mittelohr-Chirurg ohne weitere Vorbereitungen die Möglichkeit hat, unmittelbar bei der Operation -insbesondere nach Inaugenscheinnahme der konkreten individuellen Situation im Mittelohr des Patientenkurzfristig vor dem Einsetzen der Prothese eine von den beiden mit dem erfindungsgemäßen Gebinde angebotenen, unterschiedlichen Konfigurationen der Gehörknöchelchenprothese auszuwählen, um den Patienten optimal zu versorgen.

Den nicht-ausgewählten, also verworfenen Teil des Gebindes kann der Chirurg an dem durch die Kopfplatte (erstes Befestigungselement) hindurch verlängerten Schaftstück mit dem daran anhängenden nicht-gewünschten dritten Befestigungselement oberhalb der Kopfplatte ganz einfach abtrennen, beispielsweise durch simples Abbrechen oder mittels eines Separations-Werkzeugs.

Damit bleibt dann die Gehörknöchelchenprothese mit der vom Chirurgen ausgewählten Konfiguration von Kopfplatte, Verbindungselement und dem daran anhängenden gewünschten zweiten Befestigungselement als komplette und unmittelbar verwendbare Gehörknöchelchenprothese übrig und steht für die Implantation ohne weitere Anpassungen sofort zur Verfügung.

Falls das erfindungsgemäße Gebinde ein vom zweiten Befestigungselement funktional unterschiedliches drittes Befestigungselement enthält, kann also der Chirurg ohne Zeitverzögerung das für die vorgefundene tatsächliche Situation im Mittelohr des Patienten optimale zweite Befestigungselement auswählen und nach dem Entfernen des dritten Befestigungselements sofort implantieren.

Wenn das erfindungsgemäße Gebinde hingegen (zusätzlich zur immer vorhandenen Kopfplatte) zwei funktional gleichartige Befestigungselemente enthält, aber die Schaftlänge zwischen Kopfplatte und dem zweiten Befestigungselement verschieden ist von der Schaftlänge zwischen Kopfplatte und dem dritten Befestigungselement, kann der Chirurg auf einfache Weise eine Einstellung der gewünschten Prothesen-Länge durch seine entsprechende Auswahl vornehmen.

In jedem Fall gewährt die vorliegende Erfindung dem behandelnden Arzt eine deutlich größere Flexibilität bei der Mittelohr-Operation.

Zudem wird die Notwendigkeit einer gleichzeitigen Lagerhaltung von vielen unterschiedlichen Mittelohrprothesen erheblich verringert.

### Bevorzugte Ausführungsformen der Erfindung

Das erfindungsgemäße Gebinde mit einem Schaft-förmigen Verbindungselement sowie einer flachen Kopfplatte zur mechanischen Anlage am Trommelfell als zentrale Bestandteile sowie einem zweiten Befestigungselement an einem Ende des Schaftes und einem dritten Befestigungselement am gegenüberliegenden Schaftende kommt prinzipiell ohne weitere "Zutaten" aus.

Um aber das Abtrennen des nicht-ausgewählten Teiles des Gebindes mittels Abbrechen zu erleichtern, zeichnet sich eine besonders bevorzugte Klasse von Ausführungsformen der erfindungsgemäßen Gehörknöchelchenprothese dadurch aus, dass das Schaft-förmige Verbindungselement in seinen beiden an die Durchgangsbohrung des ersten Befestigungselements beiderseits angrenzenden Bereichen jeweils eine Sollbruchstelle aufweist.

Bei einer bevorzugten Weiterbildung dieser Klasse von Ausführungsformen umfasst die Sollbruchstelle um das Schaft-förmige Verbindungselement Ring-förmig azimutal umlaufende Materialausnehmung.

Eine solche Sollbruchstelle lässt sich etwa durch Drehen oder Einfräßen der ringförmigen Materialausnehmung erzeugen und erfordert beim Abknicken der nicht-gewünschten Teils der Prothese keine besondere Auswahl der Abknick-Richtung, sondern das Abknicken kann bezüglich der Schaftachse in jeder beliebigen radialen Richtung erfolgen.

Alternativ dazu ist bei einer anderen Weiterbildung vorgesehen, dass eine Kerben- oder Keil-förmige, einseitige radiale Materialausnehmung im Schaft-förmigen Verbindungselement als Sollbruchstelle ober- und unterhalb des Schaft-förmigen Verbindungselements angebracht ist. Eine solche Kerbe oder ein Keil kann technisch äußerst einfach und unaufwändig seitlich am Schaft hergestellt werden, gibt dann allerdings eine bevorzugte radiale Richtung beim Abknicken vor.

Eine erste Gruppe von Ausführungsformen des erfindungsgemäßen Gebindes zeichnet sich dadurch aus, dass das erste Befestigungselement zusammen mit dem zweiten Befestigungselement sowie mit einem dazwischen befindlichen ersten Teil des Schaft-förmigen Verbindungselements eine Total-Prothese bildet, während das erste Befestigungselement zusammen mit dem dritten Befestigungselement sowie mit einem dazwischen befindlichen zweiten Teil des Schaftförmigen Verbindungselements eine Partial-Prothese bildet.

Ein Vorteil dieser Konfiguration besteht darin, dass der Mittelohr-Chirurg erst unmittelbar vor dem Einsetzen der endgültigen Mittelohrprothese endgültig entscheiden muss, ob angesichts der vorgefundenen tatsächlichen Situation im Mittelohr des Patienten eine Total-Prothese erforderlich ist, welche sämtliche Teile der Gehörknöchelchen überbrückt, oder ob noch ausreichende Substanz bei einem oder mehreren Gehörknöchelchen vorhanden ist, um eine Partial-Prothese einzusetzen.

Bei einer zweiten Gruppe von Ausführungsformen der Erfindung bildet das erste Befestigungselement zusammen mit dem zweiten Befestigungselement sowie mit einem dazwischen befindlichen ersten Teil des Schaft-förmigen Verbindungselements eine Partial-Prothese, während das erste Befestigungselement zusammen mit dem dritten Befestigungselement sowie mit einem dazwischen befindlichen zweiten Teil des Schaft-förmigen Verbindungselements ebenfalls eine Partial-Prothese bildet.

Damit ist beim Einsatz dieses Gebindes zwar bereits entschieden, dass die zu implantierende Gehörknöchelchenprothese eine Partial-Prothese sein wird. Der behandelnde Chirurg kann hier aber noch die Art des zweiten Befestigungselements kurzfristig auswählen.

Wenn -etwa durch Voruntersuchungen des Patienten- bereits feststeht, dass die Gehörknöchelchen bereits dermaßen geschädigt sind, dass eine Partial-Prothese nicht mehr in Betracht kommt, sondern eine Total-Prothese eingesetzt werden muss, kann der behandelnde Chirurg bei eine dritten Gruppe von Ausführungsformen des erfindungsgemäßen Gebindes immerhin noch auswählen, welcher Art das ausgewählte zweite Befestigungselement sein soll:
Hier bildet nämlich das erste Befestigungselement zusammen mit dem zweiten Befestigungselement sowie mit einem dazwischen befindlichen ersten Teil des Schaft-förmigen Verbindungselements eine Total-Prothese, und das erste Befestigungselement zusammen mit dem dritten Befestigungselement sowie mit einem dazwischen befindlichen zweiten Teil des Schaft-förmigen Verbindungselements bildet ebenfalls eine Total-Prothese.

Vorteilhaft sind Weiterbildungen der oben beschriebenen zweiten und dritten Gruppe von Ausführungsformen, die sich dadurch auszeichnen, dass der erste Teil des Schaft-förmigen Verbindungselements zwischen dem ersten Befestigungselement und dem zweiten Befestigungselement eine unterschiedliche axiale Länge gegenüber dem zweiten Teil des Schaft-förmigen Verbindungselements zwischen dem ersten und dem dritten Befestigungselement aufweist.

Auf diese Weise kann der behandelnde Chirurg, wenn schon feststeht, ob eine Partial- oder eine Total-Prothese eingesetzt werden muss, noch unkompliziert die ungefähre Länge derselben grob vorwählen.

Diese Weiterbildungen lassen sich in ihrer Praxistauglichkeit noch dadurch verbessern, dass der erste Teil des Schaft-förmigen Verbindungselements eine axiale Länge zwischen 1,75mm und 3,5mm, vorzugsweise 2mm, aufweist, und dass der zweite Teil des Schaftförmigen Verbindungselements eine axiale Länge zwischen 3mm und 7mm, vorzugsweise 4mm, aufweist.

In der Praxis bewähren sich auch Ausführungsformen der erfindungsgemäßen Gehörknöchelchenprothese, bei welchen as Schaft-förmige Verbindungselement eine radiale Dicke quer zur Längsachse zwischen 0,1mm und 0,2mm, vorzugsweise 0,18mm, aufweist.

Durch die Auswahl der genauen Art des gewünschten zweiten (beziehungsweise dritten) Befestigungselements legt der behandelnde Chirurg die optimale Ausführung der verwendeten Gehörknöchelchenprothese für den jeweiligen Patienten fest.

So sind etwa Ausführungsformen des erfindungsgemäßen Gebindes dadurch gekennzeichnet, dass das zweite Befestigungselement und/oder das dritte Befestigungselement als geschlitzte Glocke ausgebildet ist.

Weitere vorteilhafte Ausführungsformen der Erfindung sind dadurch gekennzeichnet, dass das zweite Befestigungselement und/oder das dritte Befestigungselement als Clip ausgebildet ist.

Vorteilhaft können auch Ausführungsformen des erfindungsgemäßen Gebindes sein, bei welchen das zweite Befestigungselement und/oder das dritte Befestigungselement Stempel-förmig ausgebildet ist.

Möglich und unter Umständen bevorzugt sind auch Ausführungsformen, bei welchen das zweite Befestigungselement und/oder das dritte Befestigungselement als Piston ausgebildet ist.

Die besonderen Wirkungsweisen und Vorteile dieser oben genannten vier Varianten sind in der Detail-Beschreibung unten näher erläutert.

Zusammenfassend kann also bei Ausgestaltungen der Erfindung das zweite Befestigungselement als Platte, insbesondere als gebogene Platte, als Hülse, als Schlinge, als geschlossene Glocke, insbesondere in Form eines Hohlzylinders, als einfach oder mehrfach geschlitzte Glocke oder als Clip zur mechanischen Verbindung mit einem weiteren Glied der Gehörknöchelchenkette ausgebildet sein. Alternative Ausführungsformen können vorsehen, dass die Gehörknöchelchenprothese an ihrem das zweite Befestigungselement tragenden Ende mittels Perforation der Steigbügelfußplatte (=Stapedektomie bzw. Stapedotomie) und/oder mittels Eröffnung der menschlichen Hörschnecke (=Cochleotomie) direkt an das Innenohr angekoppelt ist, insbesondere über einen Kolben.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager" dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein zeitlich lang andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausgestaltung der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen. Die Kopfplatte der erfindungsgemäßen Gehörknöchelchenprothese sollte grundsätzlich eine wachstumsfördernde Beschichtung, ein direkt ins Innenohr führendes, etwa in Form eines Kolbens ausgebildetes zweites Befestigungselement hingegen eher eine wachstumshemmende Beschichtung aufweisen.

Durch die Verwendung von biokompatiblen Materialien wie Gold oder Titan, die oft in Gehörknöchelchenprothesen verwendet werden, wird das Risiko von postoperativen Infektionen und Abstoßungsreaktionen reduziert.

Alternativ oder ergänzend können bei weiteren Ausführungsformen Teile der erfindungsgemäßen Gehörknöchelchenprothese aus einem Keramikmaterial hergestellt sein. Möglich sind aber auch Ausführungsformen der Erfindung, bei denen die Prothese oder Teile davon aus biokompatiblen Kunststoffen, insbesondere Silikon, Polytetrafluorethylen (PTFE) oder Faserverbund-werkstoffen hergestellt sind. Mit diesen Materialien können postoperative Abstoßungsreaktionen in den meisten Fällen ebenfalls verhindert werden.

In den Rahmen der vorliegenden Erfindung fällt auch ein System umfassend eine erfindungsgemäß aufgebaute Gehörknöchelchenprothese der oben beschriebenen Art sowie ein Separations-Werkzeug zum Durchtrennen des Schaft-förmigen Verbindungselements.

Das Separations-Werkzeug kann als Schneide-Zange zur Ausbildung einer definierten Spitze an der Trennstelle des gestaltet sein. Mit ihrer Hilfe entfernt der Operateur den überstehenden Prothesen-Schaft mit dem nicht-ausgewählten dritten Befestigungselement an der lateralen Seite der Trommelfell-Kopfplatte. Es entsteht ein Spike oder Pin, der bei eingesetzter Gehörknöchelchenprothese das bei der Operation zwischen Implantat und Trommelfell gelegte Transplantat-Material, wie etwa Knorpel oder Faszie, fixiert.

Das Separations-Werkzeug kann dabei als Implantationshilfe dienen, insbesondere für eine Längeneinstellung der ausgewählten Gehörknöchelchenprothese. Es kann als minimalinvasives, insbesondere endoskopisches, Instrument, vorzugsweise Pinzetten-artig oder Zangenartig, ausgebildet sein.

### Detaillierte Beschreibung der Erfindung anhand der Zeichnung

In der schematischen Zeichnung sind Ausführungsbeispiele der Erfindung dargestellt, welche in der nachfolgenden Beschreibung näher erläutert werden.

Es zeigen:
- Fig. 1a: eine schematische Seitenansicht einer ersten Ausführungsform des erfindungsgemäßen Gebindes umfassend ein durch das als Trommelfell-Kopfplatte ausgebildete erste Befestigungselement hindurchragendes Schaft-förmiges Verbindungselement mit einem als geschlitzte Glocke ausgeführten zweiten Befestigungselement an seinem einen Ende und einem als Stempel ausgeführten dritten Befestigungselement an seinem anderen Ende, wobei der erste Teil des Schafts zwischen Glocke und Kopfplatte axial gleich lang ist wie der zweite Teil des Schafts zwischen Stempel und Kopfplatte;
- Fig. 1b: eine räumliche Darstellung der Ausführungsform nach Fig. 1a, wobei der erste Teil des Schafts zwischen Glocke und Kopfplatte axial deutlich kürzer ist als der zweite Teil des Schafts zwischen Stempel und Kopfplatte;
- Fig. 1c: eine räumliche Darstellung der Ausführungsform nach Fig. 1a, wobei der erste Teil des Schafts zwischen Glocke und Kopfplatte axial deutlich länger ist als der zweite Teil des Schafts zwischen Stempel und Kopfplatte;
- Fig. 2a: eine schematische Seitenansicht einer weiteren Ausführungsform des erfindungsgemäß gestalteten Gebindes mit einem als geschlitzte Glocke ausgeführten zweiten Befestigungselement am einen Ende des Schaft-förmigen Verbindungselements und einem als Clip ausgeführten dritten Befestigungselement an dessen anderem Ende, wobei die beiden Schaft-Teile zwischen Glocke und Kopfplatte sowie zwischen Kopfplatte und Clip axial gleich lang sind;
- Fig. 2b: eine räumliche Darstellung der Ausführungsform nach Fig. 2a, wobei der erste Teil des Schafts zwischen Glocke und Kopfplatte axial deutlich kürzer ist als der zweite Teil des Schafts zwischen Clip und Kopfplatte;
- Fig. 2c: eine räumliche Darstellung der Ausführungsform nach Fig. 2a, wobei der erste Teil des Schafts zwischen Glocke und Kopfplatte axial deutlich länger ist als der zweite Teil des Schafts zwischen Clip und Kopfplatte;
- Fig. 3: eine Ausführungsform eines erfindungsgemäß gestalteten Gebindes, bei der sowohl das zweite als auch das dritte Befestigungselement jeweils als geschlitzte Glocke ausgeführt ist, wobei die beiden Teile des Schafts zwischen der Kopfplatte und den beiden Glocken unterschiedlich lang sind;
- Fig. 4: eine Ausführungsform eines erfindungsgemäß gestalteten Gebindes, bei der sowohl das zweite als auch das dritte Befestigungselement jeweils als Clip ausgeführt ist, wobei die beiden Teile des Schafts zwischen der Kopfplatte und den beiden Clips unterschiedlich lang sind;
- Fig. 5: eine Ausführungsform eines erfindungsgemäß gestalteten Gebindes, bei der sowohl das zweite als auch das dritte Befestigungselement jeweils als Stempel ausgeführt ist, wobei die beiden Teile des Schafts zwischen der Kopfplatte und den beiden Stempeln unterschiedlich lang sind;
- Fig. 6: eine Ausführungsform eines erfindungsgemäß gestalteten Gebindes, bei der sowohl das zweite als auch das dritte Befestigungselement jeweils als Piston ausgeführt ist, wobei die beiden Teile des Schafts zwischen der Kopfplatte und den beiden Pistons unterschiedlich lang sind und die beiden Pistons unterschiedliche radiale Durchmesser aufweisen;
- Fig. 7a: eine Ausführungsform des erfindungsgemäß gestalteten Gebindes ähnlich wie in Fig. 2a, aber mit einer den Schaft jeweils oberhalb und unterhalb der Kopfplatte Ring-förmig azimutal umlaufenden Materialausnehmung als Sollbruchstellen;
- Fig. 7b: eine Ausführungsform ähnlich wie in Fig. 7a, jedoch mit einer Kerben- oder Keil-förmigen, einseitig radialen Materialausnehmung im Schaft jeweils oberhalb und unterhalb der Kopfplatte als Sollbruchstellen;
- Fig. 8: eine räumliche Detail-Ansicht des als Kopfplatte ausgestalteten ersten Befestigungselements mit durchgehendem Schaft-förmigen Verbindungselement oberhalb und unterhalb der Kopfplatte;
- Fig. 9: eine schematische seitliche Darstellung einer Ausführungsform des erfindungsgemäßen Gebindes *nach* Abtrennen des nicht-ausgewählten Gebinde-Teils und Ausbildung einer Spike-förmigen Spitze am Schaft im Bereich der dem ausgewählten Teil (hier mit Stempel-förmigem Befestigungselement) gegenüberliegenden Seite der Kopfplatte; und
- Fig. 10: eine schematische Seitenansicht eines als Schneide-Zange geformten Separations-Werkzeugs zum Durchtrennen des Schaft-förmigen Verbindungselements und zur Ausbildung einer geometrisch definierten Spitze an der Trennstelle zwischen dem ausgewählten Teil des Gebindes und dem nicht-ausgewählten Gebinde-Teil.

Die in den Figuren der Zeichnung schematisch dargestellten Ausführungsformen der erfindungsgemäßen **Gehörknöchelchenprothese 10; 20; 30; 40; 50; 60** (oder Teilen davon) weisen jeweils ein **erstes Befestigungselement 11; 21; 31; 41; 51; 61** auf, welches in Form einer ebenen Kopfplatte zur mechanischen Anlage am Trommelfell ausgebildet ist. Am einen Ende der Gehörknöchelchenprothese 10; 20; 30; 40; 50; 60 sitzt jeweils ein **zweites Befestigungselement 12; 22; 32; 42; 52; 62** zur mechanischen Verbindung der Prothese mit einem Glied oder Teilen eines Gliedes der Gehörknöchelchen-Kette oder mit dem Innenohr. Dazwischen ist ein entlang einer **Längsachse z** die beiden Befestigungselemente 11,12; 21,22; 31,32; 41,42 Schall-leitend miteinander verbindendes, Schaft-förmiges **Verbindungselement 14; 24; 34; 44; 54; 64** angeordnet, wobei das Platten-förmige erste Befestigungselement 11; 21; 31; 41; 51; 61 in seinem Zentrum eine **Durchgangsbohrung 15; 25** zur Aufnahme des Schaft-förmigen Verbindungselements 14; 24; 34; 44; 54; 64 aufweist, die zur Fixierung des Verbindungselements 14; 24; 34; 44; 54; 64 verengbar gestaltet ist

Die Erfindung zeichnet sich gegenüber bekannten gattungsgemäßen Gehörknöchelchenprothesen dadurch aus, dass das Schaft-förmige Verbindungselement 14; 24; 34; 44; 54; 64 durch die Durchgangsbohrung 15; 25; 35; 45 hindurch auf die dem zweiten Befestigungselement 12; 22; 32; 42; 52; 62 gegenüberliegende Seite des ersten Befestigungselements 11; 21; 31; 41; 51; 61 ragt, dass das Verbindungselement 14; 24; 34; 44; 54; 64 an seinem dem zweiten Befestigungselement 12; 22; 32; 42; 52; 62 gegenüberliegenden freien Ende ein **drittes Befestigungselement 13; 23; 33; 43; 53; 63** trägt, welches zur mechanischen Verbindung mit einem Glied oder Teilen eines Gliedes der Gehörknöchelchen-Kette oder mit dem Innenohr ausgestaltet ist, und dass das Schaft-förmige Verbindungselement 14; 24; 34; 44; 54; 64 zumindest in seinen beiden an die Durchgangsbohrung 15; 25; 35; 45 des ersten Befestigungselements 11; 21; 31; 41; 51; 61 beiderseits angrenzenden Bereichen so gestaltet ist, dass es oberhalb oder unterhalb des Platten-förmigen ersten Befestigungselements 11; 21; 31; 41; 51; 61 abgebrochen oder durch ein **Separations-Werkzeug 16** abgetrennt werden kann.

Dieses erfindungsgemäße Gebinde gibt dem behandelnden Chirurgen die Möglichkeit an die Hand, sich erst unmittelbar vor der eigentlichen Implantation der Gehörknöchelchenprothese zu entscheiden, wie diese ausgestaltet sein soll. Der nicht-ausgewählte Teil des Gebindes wird dann ganz einfach entfernt. Die axiale Länge der vom Chirurgen ausgewählten Gehörknöchelchenprothese kann beispielsweise durch Verschieben des als Trommelfell-Kopfplatte ausgebildeten ersten Befestigungselements 11; 21; 31; 41; 51; 61 längs des Schaft-förmigen Verbindungselements 14; 24; 34; 44; 54; 64 und anschließendem Befestigen oder Versteifen derselben am Schaft, etwa mittels eines Crimp-Vorgangs, eingestellt werden.

Mit dem erfindungsgemäßen Gebinde reduziert sich die notwendige Vorratshaltung unterschiedlicher Gehörknöchelchenprothesen beträchtlich. Dem Otologen bietet sich mit der vorliegenden Erfindung eine große Vielzahl von Auswahl-Varianten im Bereich der Tympanoplastik an.

Bei der Tympanoplastik gibt es verschiedene Möglichkeiten der Ankopplung an den Stapes, je nach Art und Umfang des chirurgischen Eingriffs sowie dem Zustand des Mittelohrs.

Im Folgenden sind die gängigsten Methoden aufgezählt und erläutert:
1. **Ossikuloplastik:**
   ∘ **Partielle Ossikel-Rekonstruktions-Prothese ("PORP"):**
   ∘ Hierbei wird eine prothetische Verbindung zwischen dem Trommelfell und dem Stapes-Kopf hergestellt. Dabei ist der Stapes weitestgehend intakt.

Die Verwendung einer PORP bei der Tympanoplastik bietet mehrere Vorteile:
(a)**Erhaltung der natürlichen Schallübertragung:**
   ∘ Eine PORP ermöglicht eine effektivere Schallübertragung, da sie die verbleibenden intakten Teile der Gehörknöchelchen (insbesondere den Stapes) nutzt und somit die physiologische Schallleitung weitgehend erhalten bleibt.
(b)**Bessere Anpassung und Stabilität:**
   ∘ Die PORP kann gut an die anatomischen Gegebenheiten des Mittelohrs angepasst werden, was zu einer stabileren und sichereren Verbindung führt. Dies verringert das Risiko von Prothesenluxationen oder -verschiebungen im Vergleich zu anderen Rekonstruktionsmethoden.
(c)**Schonung der Stapes-Fußplatte:**
   ∘ Durch die Verwendung einer PORP wird die Stapes-Fußplatte oft weniger belastet oder manipuliert, was das Risiko von Komplikationen wie Fußplattenluxation oder -frakturen minimiert.
(d)**Verbesserte Hörresultate:**
   ∘ Studien haben gezeigt, dass Patienten mit einer PORP tendenziell bessere postoperative Hörverbesserungen aufweisen, insbesondere im Bereich der mittleren und hohen Frequenzen, im Vergleich zu anderen Methoden wie der TORP.
(e)**Vielseitigkeit in der Anwendung:**
   ∘ Eine PORP kann in einer Vielzahl von klinischen Situationen eingesetzt werden, z.B. durch chronische Mittelohrentzündungen, wo der Stapes intakt ist.
(f) **Geringeres Infektionsrisiko:**
   ∘ Durch die Verwendung von biokompatiblen Materialien wie Titan, die oft in PORP-Prothesen verwendet werden, wird das Risiko von postoperativen Infektionen und Abstoßungsreaktionen reduziert.

Insgesamt bietet die PORP eine effektive und zuverlässige Methode zur Wiederherstellung der Schallleitung im Mittelohr, wobei die natürlichen Strukturen und Funktionen des Ohres so weit wie möglich erhalten bleiben.

2. **Totale Ossikel-Rekonstruktions-Prothese ("TORP"):** Wenn der Amboss und Malleus komplett fehlen, wird eine Prothese verwendet, die eine direkte Verbindung zwischen dem Trommelfell und der Fußplatte des Stapes herstellt.

Die Verwendung einer Totalen Ossikelrekonstruktion (TORP) bei der Tympanoplastik bietet mehrere spezifische Vorteile:
(a)**Wiederherstellung der Schallübertragung bei stark zerstörtem Ossikelketten:**
   ∘ TORP ist besonders nützlich in Fällen, in denen der Amboss und Hammer weitgehend zerstört oder entfernt werden mussten. Diese Methode ermöglicht die Wiederherstellung der Schallübertragung selbst in stark beeinträchtigten Mittelohrstrukturen.
(b)**Direkte Verbindung zwischen Trommelfell und Stapes-Fußplatte:**
   ∘ Eine TORP schafft eine direkte Verbindung zwischen dem Trommelfell und der Stapes-Fußplatte, was eine effiziente Schallübertragung ermöglicht. Dies kann besonders vorteilhaft sein, wenn die verbliebenen Teile der Gehörknöchelchen nicht mehr verwendet werden können.
(c)**Anpassung an individuelle anatomische Gegebenheiten:**
   ∘ TORP-Prothesen können in Länge und Form angepasst werden, um den spezifischen anatomischen Gegebenheiten des Patienten gerecht zu werden. Dies verbessert die Passgenauigkeit und die Stabilität der Prothese.
(d)**Reduziertes Risiko von Ossikelprothesen-Verschiebungen:**
   ∘ Die direkte und stabile Verbindung zwischen Trommelfell und Stapes-Fußplatte verringert das Risiko, dass die Prothese sich verschiebt oder lockert, was zu einer langfristig besseren Hörfunktion führt.
(e)**Umgehung beschädigter oder nicht funktionierender Ossikel:**
   ∘ Bei erheblichen Schäden oder Fehlfunktionen der natürlichen Ossikelkette kann eine TORP die beschädigten Strukturen umgehen und dennoch eine effektive Schallübertragung gewährleisten.

Eine besonders vorteilhafte Variante der TORP sieht einen ovalen Stempel für eine größere Auflagefläche vor. Die aus Titan hergestellte Totalprothese der Anmelderin vom Typ "Regensburg" besitzt durch ihr ovales und vergrößertes Stempelende einen großen Kontaktbereich zur Steigbügelfußplatte. Zusammen mit einem tieferen Schwerpunkt sorgt sie für ein Höchstmaß an Balance und erleichtert damit das intraoperative Handling.

Weitere vorteilhafte Merkmale dieser TORP: Kleine Aussparungen in der Kopfplatte indizieren die Lage des Stempels. Unterhalb der Kopfplatte hat der Schaft einen Durchmesser von nur 0,2mm. Dadurch lässt er sich leicht biegen und kann sehr gut auf die in gewünschte Endform gebracht werden.

Zusammengefasst bietet die TORP eine effektive Lösung zur Wiederherstellung der Schallleitung im Mittelohr, insbesondere in Fällen, in denen die natürliche Ossikelkette stark beeinträchtigt ist. Die Möglichkeit, eine stabile und direkte Verbindung zwischen dem Trommelfell und der Stapes-Fußplatte zu schaffen, führt häufig zu guten audiologischen Ergebnissen und einer verbesserten Lebensqualität für die Patienten.

### 3. Stapes-Fußplatten-Ankopplung:

∘ **Stapesprothese:** Diese Methode kommt bei der Stapedotomie oder Stapedektomie zum Einsatz, oft bei der Behandlung der Otosklerose. Eine Prothese wird auf der Stapes-Fußplatte verankert, entweder durch Einsetzen in eine kleine Öffnung (Stapedotomie) oder nach Entfernung der Stapes-Fußplatte (Stapedektomie).

Die Verwendung einer Stapesprothese bietet mehrere Vorteile, insbesondere bei der Behandlung von Otosklerose und anderen

Erkrankungen, die die Bewegung des Stapes behindern. Hier sind einige der wichtigsten Vorteile:
(a)**Verbesserte Hörleistung:**
   ∘ Eine Stapesprothese kann die Schallübertragung erheblich verbessern, indem sie die Steifheit oder Fixierung des Stapes umgeht und somit die mechanische Bewegung der Schallwellen vom Mittelohr zur Cochlea wiederherstellt.
(b)**Minimale Invasivität:**
   ∘ Moderne Techniken, wie die Stapedotomie, bei der nur ein kleines Loch in die Fußplatte des Stapes gebohrt wird, sind minimal invasiv und führen zu kürzeren Erholungszeiten und weniger postoperativen Beschwerden im Vergleich zu umfangreicheren Eingriffen.
(c)**Hohe Erfolgsrate:**
   ∘ Die Erfolgsraten bei Stapesprothesen sind hoch, mit einer signifikanten Anzahl von Patienten, die eine deutliche Verbesserung ihres Hörvermögens berichten. Langfristige Ergebnisse zeigen oft eine stabile Hörverbesserung.
(d)**Anpassbarkeit:**
   ∘ Stapesprothesen sind in verschiedenen Größen und Materialien erhältlich, wie z.B. Titan oder Teflon, was eine Anpassung an die spezifischen anatomischen Bedürfnisse des Patienten ermöglicht und eine optimale Passform sicherstellt.
(e)**Geringeres Risiko von Komplikationen:**
   ∘ Durch die Verwendung moderner Materialien und Techniken ist das Risiko von Komplikationen, wie Infektionen oder Abstoßungsreaktionen, gering. Zudem sind die verwendeten Materialien biokompatibel und langlebig.
(f) **Wiederherstellung der natürlichen Schallübertragung:**
   ∘ Die Stapesprothese ermöglicht eine fast natürliche Schallübertragung, was zu einer besseren Klangqualität und Sprachverständlichkeit führt. Dies ist besonders wichtig für die Lebensqualität der Patienten.
(g)**Behandlung der Otosklerose:**
   ∘ Bei der Otosklerose, einer Erkrankung, die zur Verknöcherung des Stapes führt und somit dessen Bewegung einschränkt, ist die Stapesprothese eine bewährte und effektive Behandlungsmethode, um die Schallübertragung wiederherzustellen.
(h)**Langfristige Lösung:**
   ∘ Eine gut implantierte Stapesprothese kann eine dauerhafte Lösung bieten und die Notwendigkeit weiterer chirurgischer Eingriffe reduzieren. Viele Patienten berichten von anhaltenden Verbesserungen ihres Hörvermögens über viele Jahre.
(i) **Verbesserte Lebensqualität:**
   o Durch die Wiederherstellung des Hörvermögens verbessert sich oft die Lebensqualität der Patienten erheblich. Sie können wieder besser an Gesprächen teilnehmen und soziale Isolation vermeiden, was zu einem allgemein besseren Wohlbefinden führt.

Zusammengefasst bietet die Stapesprothese eine effektive und dauerhafte Lösung zur Wiederherstellung des Hörvermögens bei Patienten mit Stapesfixation oder Otosklerose, mit hoher Erfolgsrate und geringen Risiken.

Um dem behandelnden Chirurgen eine möglichst zielführende Auswahl an Entscheidungsmöglichkeiten zu bieten, kann das erfindungsgemäße Gebinde in Varianten mit unterschiedlichen Kombinationen der Merkmale ausgebildet sein:

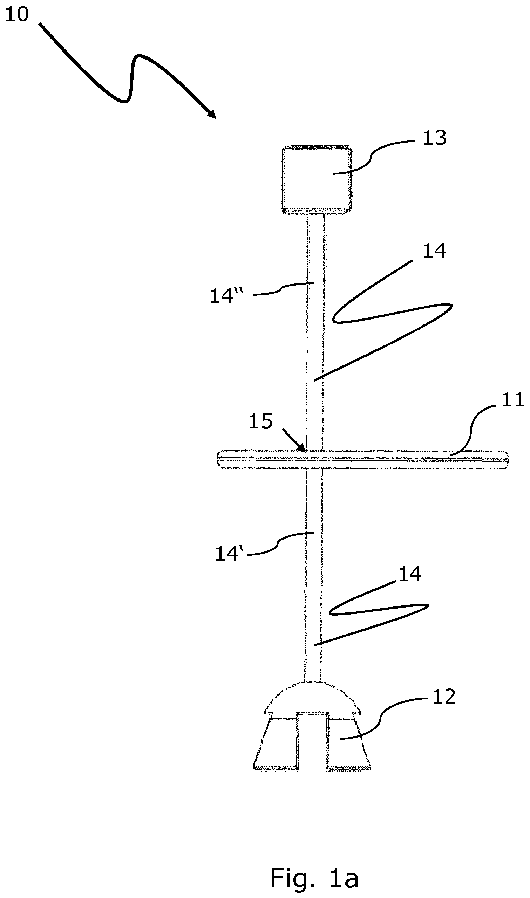

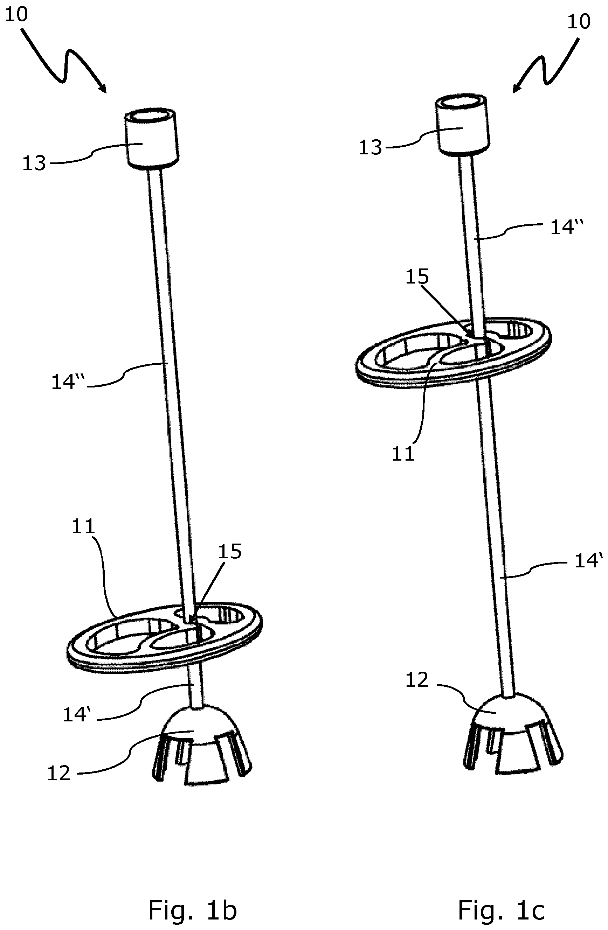

So kann -wie aus den **Figuren 1a bis 1c** zu erkennen- das erste Befestigungselement 11 zusammen mit dem zweiten Befestigungselement 12 sowie mit einem dazwischen befindlichen **ersten Teilstück 14' des Schaft-förmigen Verbindungselements** 14 eine Partial-Prothese bilden, während das erste Befestigungselement 11 zusammen mit dem dritten Befestigungselement 13 sowie mit einem dazwischen befindlichen **zweiten Teilstück 14" des Schaft-förmigen Verbindungselements** 14 eine Total-Prothese bildet.

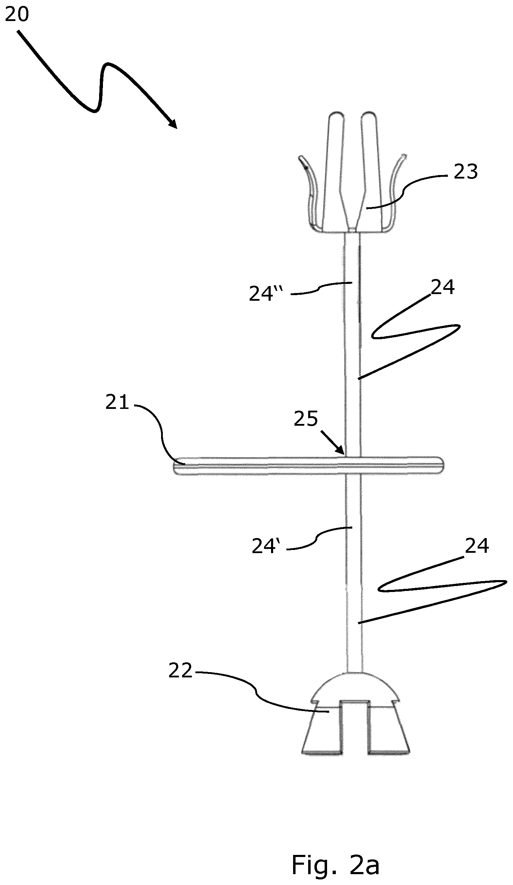

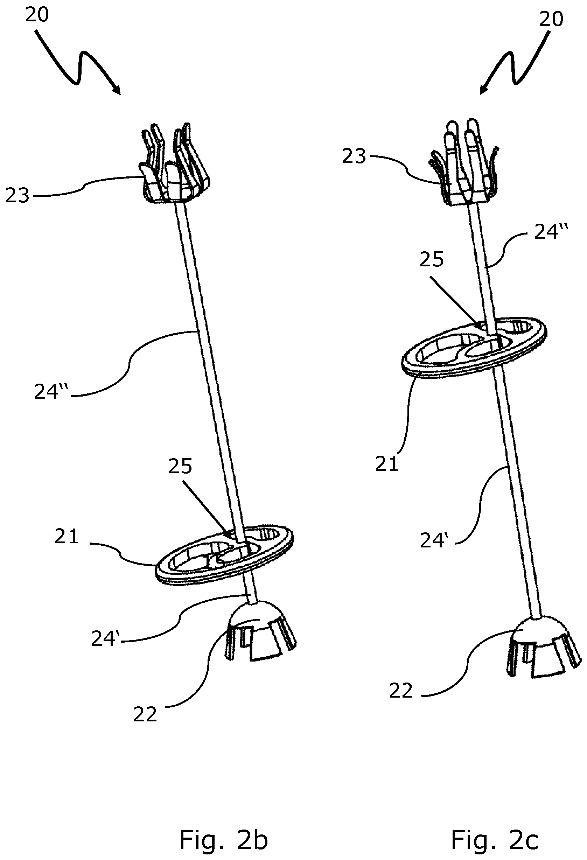

Alternativ dazu kann -wie in den **Figuren 2a bis 4** gezeigt- das erste Befestigungselement 21; 31; 41 zusammen mit dem zweiten Befestigungselement 22; 32; 42 sowie mit einem dazwischen befindlichen **ersten Teil 24'; 34'; 44'** des Schaft-förmigen Verbindungselements 24; 34; 44 eine Partial-Prothese bilden, während das erste Befestigungselement 21; 31; 41 zusammen mit dem dritten Befestigungselement 23; 33; 43 sowie mit einem dazwischen befindlichen **zweiten Teil 24"; 34"; 44"** des Schaft-förmigen Verbindungselements 24; 34; 44 ebenfalls eine Partial-Prothese bildet.

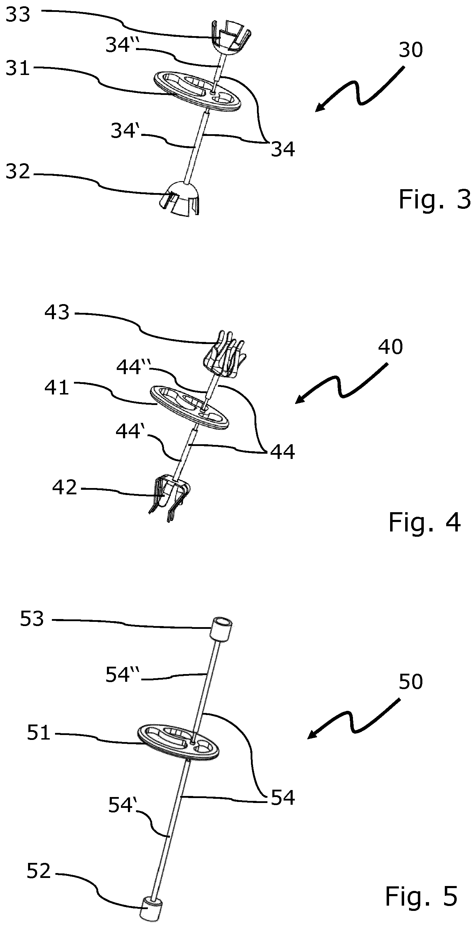

Eine weitere Variante zeigt **Fig. 5****:** Sie besteht darin, das erste Befestigungselement 51 zusammen mit dem zweiten Befestigungselement 52 sowie mit einem dazwischen befindlichen **ersten Teil 54'** des Schaft-förmigen Verbindungselements 54 eine Total-Prothese bildet, und dass das erste Befestigungselement 51 zusammen mit dem dritten Befestigungselement 53 sowie mit einem dazwischen befindlichen **zweiten Teil 54"** des Schaft-förmigen Verbindungselements 54 ebenfalls eine Total-Prothese bildet.

Bei den beiden letztgenannten Varianten der Erfindung kann es -wie in den **Figuren 1b, 1c****,** **2b, 2c** **und** **3 bis 6** gezeigt- vorteilhaft sein, wenn der erste Teil 14'; 24'; 34'; 44'; 54'; 64' des Schaft-förmigen Verbindungselements 14; 24; 34; 44; 54; 64 zwischen dem ersten Befestigungselement 11; 21; 31; 41; 51; 61 und dem zweiten Befestigungselement 12; 22; 32; 42; 52; 62 eine unterschiedliche axiale Länge gegenüber dem zweiten Teil 14"; 24"; 34"; 44"; 54"; 64" des Schaft-förmigen Verbindungselements 14; 24; 34; 44; 54; 64 zwischen dem ersten Befestigungselement 11; 21; 31; 41; 51; 61 und dem dritten Befestigungselement 13; 23; 33; 43; 53; 63 aufweist.

Auch bei der konkreten Ausgestaltung des jeweiligen zweiten Befestigungselements 12; 22; 32; 42; 52; 62 sowie des dritten Befestigungselements 13; 23; 33; 43; 53; 63 beim erfindungsgemäßen Gebinde eröffnet sich eine große Vielfalt von Variationsmöglichkeiten:
Das zweite Befestigungselement 12; 22; 32 und/oder das dritte Befestigungselement 33 kann als geschlitzte Glocke ausgebildet sein, wie in den **Figuren 1a bis 3** **sowie 7a und 7b** zu erkennen.

Die **Figuren 2a****-c und 4** zeigen jeweils Varianten, bei welchen das zweite Befestigungselement 42 und/oder das dritte Befestigungselement 23; 43 kann als Clip ausgebildet ist.

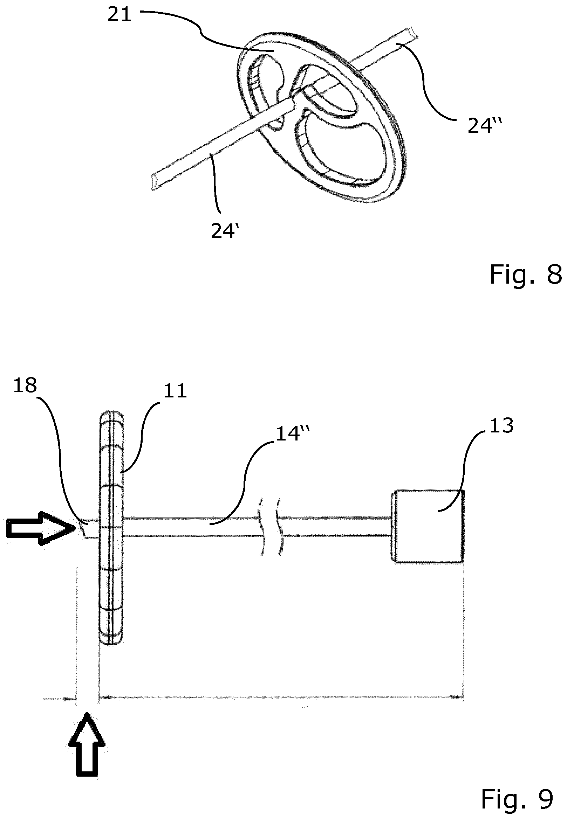

Das zweite Befestigungselement 52 und/oder das dritte Befestigungselement 13; 53 kann aber auch Stempel-förmig ausgebildet sein, wie die **Figuren 1a****-c, 5 und 9** zeigen.

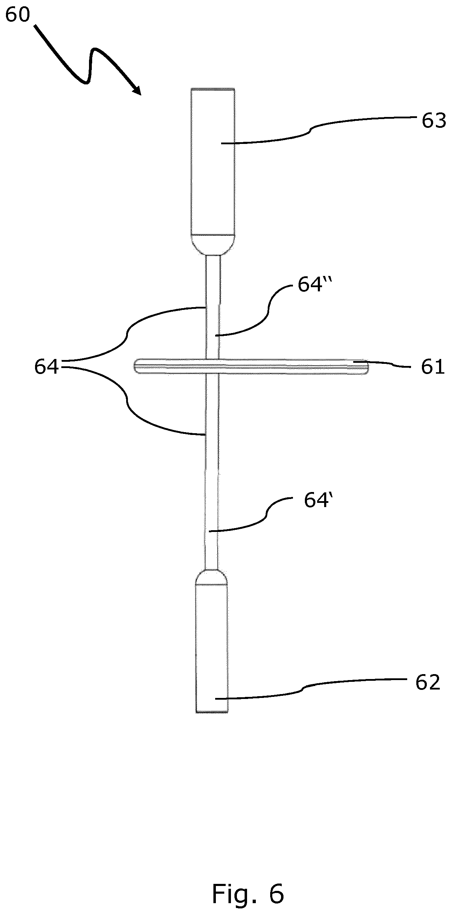

In der Ausführungsform von **Fig. 6** ist das zweite Befestigungselement 62 und auch das dritte Befestigungselement 63 sogar als Piston zur Verbindung mit dem Innenohr ausgebildet.

Wie schon oben erläutert, muss das Schaft-förmige Verbindungselement 14; 24; 34; 44; 54; 64 zumindest in seinen beiden an die Durchgangsbohrung 15; 25; 35; 45 des ersten Befestigungselements 11; 21; 31; 41; 51; 61 beiderseits angrenzenden Bereichen so gestaltet sein, dass es oberhalb oder unterhalb des Platten-förmigen ersten Befestigungselements 11; 21; 31; 41; 51; 61 leicht abgebrochen oder mittels eines Separations-Werkzeugs 16 abgetrennt werden kann.

Eine ganz einfache Gestaltungsmöglichkeit zur Gewährleistung dieser Funktion kann darin bestehen, die radiale Dicke des Schaft-förmigen Verbindungselements 14; 24; 34; 44; 54; 64 ausreichend gering zu halten. In dieser Variante kann dann die endgültige Längeneinstellung der durch den Chirurgen ausgewählten Gehörknöchelchenprothese durch einfaches Verschieben des ersten Befestigungselements 11; 21; 31; 41; 51; 61 axial längs des Schaft-förmigen Verbindungselements 14; 24; 34; 44; 54; 64 sowie anschließendes endgültiges Fest-Crimpen der Kopfplatte an der gewünschten Schaft-Position bewirkt werden.

Eine weitere Abbrech-Hilfe kann darin bestehen, dass das Schaft-förmige Verbindungselement 14; 24; 34; 44; 54; 64 in seinen beiden an die Durchgangsbohrung 15; 25; 35; 45 des ersten Befestigungselements 11; 21; 31; 41; 51; 61 beiderseits angrenzenden Bereichen jeweils eine **Sollbruchstelle 17'; 17"** aufweist.

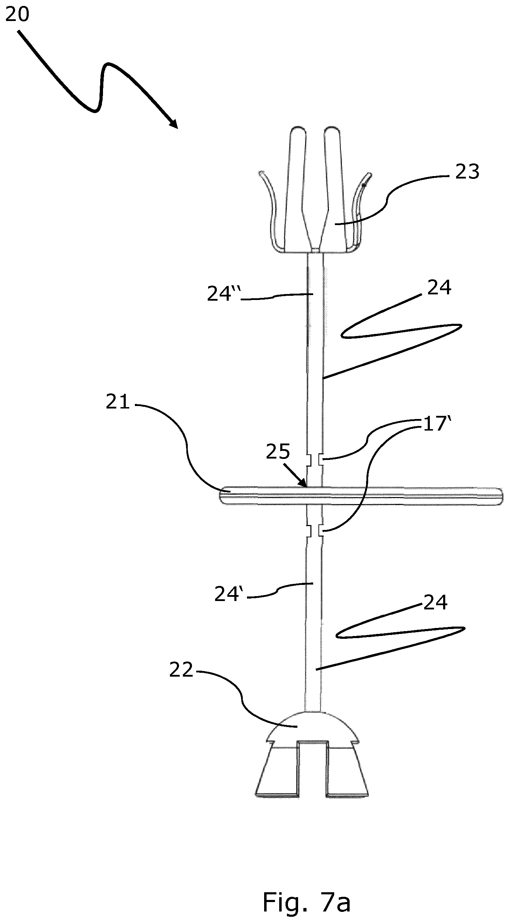

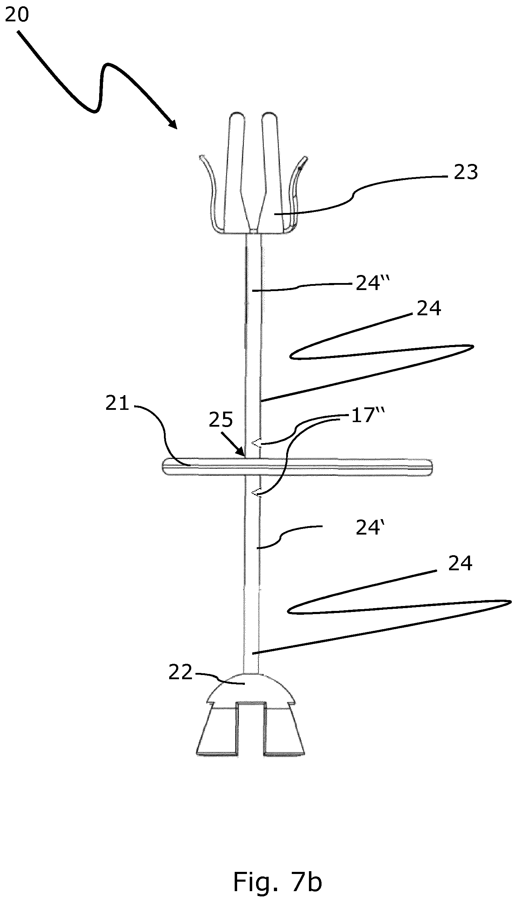

Eine solche Sollbruchstelle 17' kann eine um das Schaft-förmige Verbindungselement 14; 24; 34; 44; 54; 64 Ring-förmig azimutal umlaufende Materialausnehmung umfassen, wie in **Fig. 7a** schematisch dargestellt. Die Sollbruchstelle 17" kann aber auch als eine Kerben-oder Keil-förmige, einseitige radiale Materialausnehmung im Schaftförmigen Verbindungselement 14; 24; 34; 44; 54; 64 ausgebildet sein, in **Fig. 7b** illustriert.

Nach dem Entfernen des nicht-ausgewählten Teils des erfindungsgemäßen Gebindes durch den behandelnden Chirurgen unmittelbar vor der eigentlichen Implantation der gewünschten Gehörknöchelchenprothese liegt im ausgewählten Teil an dessen Schaftende des Verbindungselements oberhalb der Trommelfell-Kopfplatte zunächst ein Bruch- oder Schnitt-Ende vor, das vor dem Einsetzen der Prothese ins Mittelohr des Patienten entweder bearbeitet (etwa entfernt oder geglättet) werden kann. Stattdessen kann aber dieses Ende auch bewusst in seiner aktuellen Form belassen werden, insbesondere, wenn beim Entfernen des nicht-ausgewählten Teils ein Spezialwerkzeug eingesetzt wurde:

### Spike und Knorpelscheibe

Nach einer Längenanpassung der Prothese vor der Implantation und hat diese in der Regel eine maximale Länge von 0,1mm. Beim Entfernen des nicht-ausgewählten Teils des erfindungsgemäßen Gebindes (gegebenenfalls mit einem Spezialwerkzeug; siehe unten) kann bewusst eine spitze Ausformung des Bruch- oder Schnitt-Endes als "Spike" vorgenommen werden. Dieser die Trommelfell-Kopfplatte in Richtung auf das Trommelfell überragende Spike kann dann zur Fixierung einer Knorpelscheibe an die Kopfplatte (Prothesenkopf) dienen.

Bei der Verwendung von Prothesen wie der oben beschriebenen PORP oder der TORP zwischen der Kopfplatte der Prothese und dem Trommelfell kann nämlich eine Knorpelscheibe eingesetzt werden, die zuvor dem Körper des Patienten operativ entnommen wird. Diese Knorpelscheibe hat mehrere wichtige Funktionen und Vorteile:
(a)**Stabilität und Positionierung:**
   ∘ Die Knorpelscheibe hilft, die Prothese stabil zu halten und ihre Position zu sichern. Dies reduziert das Risiko von Verschiebungen oder Lockerungen der Prothese, was zu einer besseren und konsistenteren Schallübertragung führt.
(b)**Vermeidung von Trommelfellperforationen:**
   ∘ Durch das Einsetzen der Knorpelscheibe wird der direkte Kontakt zwischen der harten Kopfplatte der Prothese und dem empfindlichen Trommelfell vermieden. Dies vermindert das Risiko von Trommelfellperforationen oder Schäden, die durch mechanischen Druck oder Bewegung der Prothese entstehen könnten.
(c)**Dämpfung und Schwingungsoptimierung:**
   ∘ Die Knorpelscheibe wirkt als Dämpfungselement, das die Schwingungen zwischen der Prothese und dem Trommelfell optimiert. Dies trägt zu einer verbesserten Klangqualität bei und hilft, unangenehme Geräusche oder Verzerrungen zu vermeiden.
(d)**Biokompatibilität und Gewebeverträglichkeit:**
   ∘ Knorpel ist ein biokompatibles Material, das gut in das Gewebe des Mittelohrs integriert werden kann. Dies fördert die Heilung und reduziert das Risiko von Entzündungen oder Abstoßungsreaktionen im Vergleich zu künstlichen Materialien.
(e)**Vermeidung von Narbenbildung und Verwachsungen:**
   ∘ Die Knorpelscheibe kann helfen, die Bildung von Narbengewebe und Verwachsungen zu verhindern, die die Beweglichkeit der Prothese und die Schallübertragung beeinträchtigen könnten.
(f) **Flexibilität und Anpassung an individuelle Anatomie:**
   ∘ Knorpel kann in Größe und Form individuell angepasst werden, um eine optimale Passform und Funktion im Mittelohr zu gewährleisten. Dies ist besonders wichtig bei komplexen anatomischen Verhältnissen oder in Fällen von vorangegangenen Operationen.
(g)**Langfristige Haltbarkeit:**
   ∘ Knorpel ist ein robustes Material, das über lange Zeit hinweg stabil bleibt und nicht so leicht degeneriert oder abgebaut wird wie andere Gewebearten. Dies trägt zur langfristigen Funktionalität der rekonstruierten Ossikelkette bei.

Zusammengefasst dient die Knorpelscheibe sowohl in der PORP als auch der TORP als ein multifunktionales Element, das die Stabilität der Prothese sicherstellt, das Trommelfell schützt, die Schwingungsübertragung optimiert und die Biokompatibilität fördert. Der Spike sorgt dann für eine optimale mechanische Ankopplung der Gehörknöchelchenprothese an die Knorpelscheibe. Alle diese Faktoren tragen zu besseren chirurgischen und audiologischen Ergebnissen bei.

### Spezialwerkzeug

Auch die Verwendung eines speziell entwickelten Schneide-Zängchens kann für die fachgerechte Weiterbearbeitung der letztlich ins Mittelohr zu implantierenden Gehörknöchelchenprothese äußerst nützlich sein. Mit seiner Hilfe entfernt der Operateur den überstehenden Prothesen-Schaft an der lateralen Seite der Kopfplatte. Es entsteht ein Pin oder Spike, der bei eingesetzter Prothese das zwischen Implantat und Trommelfell eingelegte Transplantatmaterial, wie etwa Knorpel oder Faszie, fixiert.

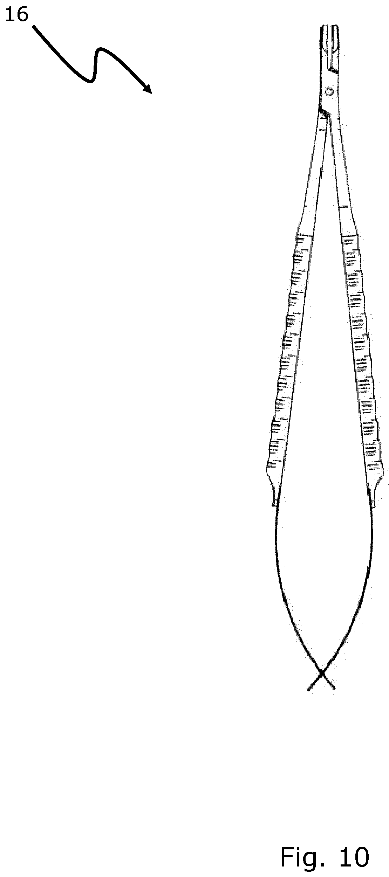

Die vorliegende Erfindung beschreibt daher auch ein System umfassend eine Gehörknöchelchenprothese 10; 20; 30; 40; 50; 60 der oben beschriebenen Art sowie ein -wie in **Fig. 10** dargestelltes- Separations-Werkzeug 16 zum Durchtrennen des Schaft-förmigen Verbindungselements 14; 24; 34; 44; 54; 64. Dieses Separations-Werkzeug 16 kann als Schneide-Zange zur Ausbildung einer geometrisch definierten Spitze an der Trennstelle des Verbindungselements 14; 24; 34; 44; 54; 64, aber auch Pinzetten-artig gestaltet sein.

### Bezugszeichenliste:

- 10; 20; 30; 40; 50; 60: Gehörknöchelchenprothese
- 11; 21; 31; 41; 51; 61: erstes Befestigungselement
- 12; 22; 32; 42; 52; 62: zweites Befestigungselement
- 13; 23; 33; 43; 53; 63: drittes Befestigungselement
- 14; 24; 34; 44; 54; 64: Verbindungselement
- 14'; 24'; 34'; 44'; 54'; 64': erstes Teilstück des Verbindungselements
- 14"; 24"; 34"; 44"; 54"; 64": zweites Teil des Verbindungselements
- 15; 25: Durchgangsbohrung
- 16: Separations-Werkzeug
- 17'; 17": Sollbruchstelle
- z: Längsachse

### Referenzliste

Für die Beurteilung der Patentfähigkeit in Betracht gezogene Publikationen:
[1] DE 298 19 892 U1 ≈ EP 0 998 884 B1 ≈ US 6,387,128 B1
[2] DE 42 10 235 C1; EP 0 809 982 B1
[3] EP 1 181 907 B1
[4] US 2004/0162614 A1
[5] EP 1 833 424 B1
[6] EP 1 972 307 B1
[7] EP 3 311 773 B1
[8] DE 10 2007 041 539 B4
[9] EP 2 238 946 B1
[10] EP 2 601 909 B1
[11] EP 3 130 315 B1
[12] EP 1 961 400 B1
[13] DE 10 2022 119 451 B3
[14] DE 10 2009 006 047 B3
[15] DE 10 2020 108 887 A1
[16] US 2020/0113675 A1

## Patentansprüche

1. Gehörknöchelchenprothese (10; 20; 30; 40; 50; 60), die mindestens ein Glied oder Teile eines Gliedes der Gehörknöchelchenkette ersetzt oder überbrückt, wobei die Gehörknöchelchenprothese (10; 20; 30; 40; 50; 60) ein als flache Kopfplatte zur mechanischen Anlage am Trommelfell ausgebildetes erstes Befestigungselement (11; 21; 31; 41; 51; 61), ein zweites Befestigungselement (12; 22; 32; 42; 52; 62) zur mechanischen Verbindung mit einem Glied oder Teilen eines Gliedes der Gehörknöchelchen-Kette oder mit dem Innenohr sowie ein entlang einer Längsachse (z) die beiden Befestigungselemente (11,12; 21,22; 31,32; 41,42; 51,52; 61,62) Schall-leitend miteinander verbindendes, Schaft-förmiges Verbindungselement (14; 24; 34; 44; 54; 64) umfasst, wobei das Platten-förmige erste Befestigungselement (11; 21; 31; 41; 51; 61) in seinem Zentrum eine Durchgangsbohrung (15; 25) zur Aufnahme des Schaft-förmigen Verbindungselements (14; 24; 34; 44; 54; 64) aufweist, die zur Fixierung des Verbindungselements (14; 24; 34; 44; 54; 64) verengbar gestaltet ist,
**dadurch gekennzeichnet,**
**dass** das Schaft-förmige Verbindungselement (14; 24; 34; 44; 54; 64) durch die Durchgangsbohrung (15; 25) hindurch auf die dem zweiten Befestigungselement (12; 22; 32; 42; 52; 62) gegenüberliegende Seite des ersten Befestigungselements (11; 21; 31; 41; 51; 61) ragt,
**dass** das Verbindungselement (14; 24; 34; 44; 54; 64) an seinem dem zweiten Befestigungselement (12; 22; 32; 42; 52; 62) gegenüberliegenden freien Ende ein drittes Befestigungselement (13; 23; 33; 43; 53; 63) trägt, welches zur mechanischen Verbindung mit einem Glied oder Teilen eines Gliedes der Gehörknöchelchen-Kette oder mit dem Innenohr ausgestaltet ist, und dass das Schaft-förmige Verbindungselement (14; 24; 34; 44; 54; 64) zumindest in seinen beiden an die Durchgangsbohrung (15; 25) des ersten Befestigungselements (11; 21; 31; 41; 51; 61) beiderseits angrenzenden Bereichen so gestaltet ist, dass es oberhalb oder unterhalb des Platten-förmigen ersten Befestigungselements (11; 21; 31; 41; 51; 61) abgebrochen oder mittels eines Separations-Werkzeugs (16) abgetrennt werden kann.

2. Gehörknöchelchenprothese nach Anspruch 1, **dadurch gekennzeichnet, dass** das Schaft-förmige Verbindungselement (24) in seinen beiden an die Durchgangsbohrung (25) des ersten Befestigungselements (21) beiderseits angrenzenden Bereichen jeweils eine Sollbruchstelle (17'; 17") aufweist.

3. Gehörknöchelchenprothese nach Anspruch 2, **dadurch gekennzeichnet, dass** die Sollbruchstelle (17') eine um das Schaft-förmige Verbindungselement (24) Ring-förmig azimutal umlaufende Materialausnehmung umfasst.

4. Gehörknöchelchenprothese nach Anspruch 2, **dadurch gekennzeichnet, dass** die Sollbruchstelle (17") eine Kerben- oder Keil-förmige, einseitige radiale Materialausnehmung im Schaftförmigen Verbindungselement (24) umfasst.

5. Gehörknöchelchenprothese nach einem der Ansprüche 1 bis 4, **dadurch gekennzeichnet, dass** das erste Befestigungselement (11) zusammen mit dem zweiten Befestigungselement (12) sowie mit einem dazwischen befindlichen ersten Teil (14') des Schaftförmigen Verbindungselements (14) eine Partial-Prothese bildet, und dass das erste Befestigungselement (11) zusammen mit dem dritten Befestigungselement (13) sowie mit einem dazwischen befindlichen zweiten Teil (14") des Schaft-förmigen Verbindungselements (14) eine Total-Prothese bildet.

6. Gehörknöchelchenprothese nach einem der Ansprüche 1 bis 4, **dadurch gekennzeichnet, dass** das erste Befestigungselement (21; 31; 41) zusammen mit dem zweiten Befestigungselement (22; 32; 42) sowie mit einem dazwischen befindlichen ersten Teil (24'; 34'; 44') des Schaft-förmigen Verbindungselements (24; 34; 44) eine Partial-Prothese bildet, und dass das erste Befestigungselement (21; 31; 41) zusammen mit dem dritten Befestigungselement (23; 33; 43) sowie mit einem dazwischen befindlichen zweiten Teil (24"; 34"; 44") des Schaft-förmigen Verbindungselements (24; 34; 44) ebenfalls eine Partial-Prothese bildet.

7. Gehörknöchelchenprothese nach einem der Ansprüche 1 bis 4, **dadurch gekennzeichnet, dass** das erste Befestigungselement (51) zusammen mit dem zweiten Befestigungselement (52) sowie mit einem dazwischen befindlichen ersten Teil (54') des Schaftförmigen Verbindungselements (54) eine Total-Prothese bildet, und dass das erste Befestigungselement (51) zusammen mit dem dritten Befestigungselement (53) sowie mit einem dazwischen befindlichen zweiten Teil (54") des Schaft-förmigen Verbindungselements (54) ebenfalls eine Total-Prothese bildet.

8. Gehörknöchelchenprothese nach einem der Ansprüche 6 oder 7, **dadurch gekennzeichnet, dass** der erste Teil (14'; 24'; 34'; 44'; 54'; 64') des Schaft-förmigen Verbindungselements (14; 24; 34; 44; 54; 64) zwischen dem ersten Befestigungselement (11; 21; 31; 41; 51; 61) und dem zweiten Befestigungselement (12; 22; 32; 42; 52; 62) eine unterschiedliche axiale Länge gegenüber dem zweiten Teil (14"; 24"; 34": 44"; 54"; 64") des Schaftförmigen Verbindungselements (14; 24; 34; 44; 54; 64) zwischen dem ersten Befestigungselement (11; 21; 31; 41; 51; 61) und dem dritten Befestigungselement (13; 23; 33; 43; 53; 63) aufweist.

9. Gehörknöchelchenprothese nach Anspruch 8, **dadurch gekennzeichnet, dass** der erste Teil (14'; 24'; 34'; 44'; 54'; 64') des Schaft-förmigen Verbindungselements (14; 24; 34; 44; 54; 64) eine axiale Länge zwischen 1,75mm und 3,5mm, vorzugsweise 2mm, aufweist, und dass der zweite Teil (14"; 24"; 34"; 44"; 54"; 64") des Schaft-förmigen Verbindungselements (14; 24; 34; 44; 54; 64) eine axiale Länge zwischen 3mm und 7mm, vorzugsweise 4mm, aufweist.

10. Gehörknöchelchenprothese nach einem der vorhergehenden Ansprüche, **dadurch gekennzeichnet, dass** das Schaft-förmige Verbindungselement (14; 24; 34; 44; 54; 64) eine radiale Dicke quer zur Längsachse (z) zwischen 0,1mm und 0,2mm, vorzugsweise 0,18mm, aufweist.

11. Gehörknöchelchenprothese nach einem der vorhergehenden Ansprüche, **dadurch gekennzeichnet, dass** das zweite Befestigungselement (12; 22; 32) und/oder das dritte Befestigungselement (33) als geschlitzte Glocke ausgebildet ist.

12. Gehörknöchelchenprothese nach einem der vorhergehenden Ansprüche, **dadurch gekennzeichnet, dass** das zweite Befestigungselement (42) und/oder das dritte Befestigungselement (23; 43) als Clip ausgebildet ist.

13. Gehörknöchelchenprothese nach einem der vorhergehenden Ansprüche, **dadurch gekennzeichnet, dass** das zweite Befestigungselement (52) und/oder das dritte Befestigungselement (13; 53) Stempel-förmig ausgebildet ist.

14. Gehörknöchelchenprothese nach einem der vorhergehenden Ansprüche, **dadurch gekennzeichnet, dass** das zweite Befestigungselement (62) und/oder das dritte Befestigungselement (63) als Piston ausgebildet ist.

15. System umfassend eine Gehörknöchelchenprothese (10; 20; 30; 40; 50; 60) nach einem der vorhergehenden Ansprüche, sowie ein Separations-Werkzeug (16) zum Durchtrennen des Schaftförmigen Verbindungselements (14; 24; 34; 44; 54; 64), **dadurch gekennzeichnet, dass** das Separations-Werkzeug (16) als Schneide-Zange zur Ausbildung einer geometrisch definierten Spitze an der Trennstelle des Verbindungselements (14; 24; 34; 44; 54; 64), vorzugsweise auch Pinzetten-artig, gestaltet ist.
